# EUROPEAN PATENT APPLICATION

(11) **EP 1 847 243 A1**
(43) Date of publication of application: **24.10.2007**
(21) Application number: 06711736.6
(22) Date of filing: 16.01.2006
(51) Int. Cl.: A61K 6/00

(54) **O/W EMULSION COMPOSITION**

(30) Priority: 17.01.2005 JP 2005008508
(71) Applicant: TAISHO PHARMACEUTICAL CO., LTD, Tokyo 170-8633 (JP)
(72) Inventor: NISHIOKU, Yoshinori, 3-chome, Toshima-ku Tokyo, 1708633 (JP); SUZUKI, Yasuyuki, 3-chome, Toshima-ku Tokyo, 1708633 (JP); KONDO, Osamu, 3-chome, Toshima-ku Tokyo, 1708633 (JP); MARUMOTO, Aya, 3-chome, Toshima-ku Tokyo, 1708633 (JP)
(74) Representative: HOFFMANN EITLE
(86) International application number: PCT/JP2006/300455
(87) International publication number: WO 2006/075743

(57) **Abstract**

The present invention provides an O/Wemuls ion composition which stimulates little when used on the skin, is safe, can be preserved stably over a long period of time and has excellent feel when used by adding 40 wt% or less of a specific oil component relative to the total amount of the composition to partially saponified polyvinyl alcohol of which a degree of saponification is 70 to 96 mol% and a carboxyvinyl polymer and adjusting the pH to 3.5 to 8.5. This can be used in the same form as generally used agents, such as creams, lotions, ointments, gels and sprays.

## Description

### TECHNICAL FIELD

The present invention relates to an O/W emulsion composition which has little stimulation to the skin, is safe, can be preserved stably over a long period of time, and has excellent feel when used.

### BACKGROUND ART

Conventionally, a surfactant has been generally used to stably preserve conventional emulsion compositions.

In emulsion compositions to be used directly on the skin, such as those for pharmaceutical products and cosmetics, however, the surfactant becomes a cause of the skin being stimulated (see Non-Patent Documents 1 and 2).

Accordingly, manufacture of an emulsion composition into which no surfactant is mixed has been attempted, in order to provide a safer emulsion composition.

Known examples of such an emulsion composition include an emulsion composition using a modified polyvinyl alcohol obtained by saponifying a copolymer of alkaline olefin sulfonate and vinyl acetate (see Patent Document 1) and an emulsion composition using an alkyl modified carboxyvinyl polymer such as acrylate methacrylate alkyl copolymer (see Patent Document 2).

However, the purpose of use of the above-described component may be limited, that is, in some cases, use for medical and pharmaceutical products is not permitted.

In addition, a cream obtained by mixing a polyvinyl alcohol, a carboxyvinyl polymer and a surfactant, the cream having excellent massaging effects and blood circulation stimulating effects, has been disclosed as a massage cosmetic for under the eyes (see Patent Document 3).

However, a surfactant is mixed into the above-described cream, and therefore, an emulsion composition made of partially saponified polyvinyl alcohol and a carboxyvinyl polymer which is stable even with no surfactant mixed therein has yet to be obtained.

[Non-Patent Document 1] Tamie Suzuki et al., Japanese Journal of Dermatology, 51, 177-183 (2004)
[Non-Patent Document 2] Hisashi Tatsumi et al., Skin, 33 (11), 31-38 (1991)
[Patent Document 1] Japanese Laid-Open patent publication No. 51-3383
[Patent Document 2] Japanese Laid-Open patent publication No. 09-19631
[Patent Document 3] Japanese Laid-Open patent publication No. 2001-39851

### DISCLOSURE OF THE INVENTION

### PROBLEM TO BE SOLVED BY THE INVENTION

An object of the present invention is to provide an O/W emulsion composition where partially saponified polyvinyl alcohol and a carboxyvinyl polymer are used with substantially no surfactant mixed therein, and which can be preserved stably over a long period of time and has excellent feel when used.

### MEANS FOR SOLVING PROBLEM

The present inventors have diligently conducted research in order to solve these problems, and as a result, have found that an O/W emulsion composition which has little stimulation to the skin, is safe, can be preserved stably over a long period of time and spreads lightly to provide excellent feel when used, can be obtained by adding 40 wt% or less of a specific oil component relative to the total amount of the composition to partially saponified polyvinyl alcohol of which a degree of saponification is 70 to 96 mol% and a carboxyvinyl polymer and adjusting the pH to 3.5 to 8.5, and thus completed the present invention.

More specifically, the present invention is the following.
(1) An O/W emulsion composition, containing:
   a) partially saponified polyvinyl alcohol of which a degree of saponification is 70 to 96 mol%;
   b) a carboxyvinyl polymer; and
   c) 40 wt% or less of an oil component relative to the total amount of the composition, where 60 wt% or more of the oil component is a liquid oil, wherein
   d) the pH is 3.5 to 8.5.
(2) The 0/W emulsion composition according to the above (1), including substantially no surfactant.
(3) The O/W emulsion composition according to the above (1), wherein the liquid oil is polar liquid oil.
(4) The O/W emulsion composition according to the above (3), wherein the polar liquid oil is synthetic oil of which an IOB value is 0.1 or more.
(5) The O/W emulsion composition according to the above (3), wherein the polar liquid oil is a vegetable oil of which a main component is triglyceride fatty acid.
(6) The O/W emulsion composition according to any one of the above (1) to (5), which is a composition for external application.
(7) The O/W emulsion composition according to any one of the above (1) to (6), into which a polyol is further is mixed.

### ADVANTAGE OF THE INVENTION

The present invention has succeeded in obtaining a highly safe O/W emulsion composition which can be preserved stably over a long period of time and has little stimulation to the skin. Furthermore, the present invention makes it possible to obtain an O/W emulsion composition which spreads lightly with excellent feel continuing when used, even after application.

### BEST MODE FOR CARRYING OUT THE INVENTION

The term "partially saponified polyvinyl alcohol" in the present invention means partially saponified polyvinyl acetate obtained by polymerizing vinyl acetate; there are several types having different viscosities, such that the viscosity of a 4% aqueous solution is, for example, 4.8 to 5.8 mPa·s, 20.5 to 24.5 mPa·s, 27.0 to 33.0 mPa·s, 40.0 to 46.0 mPa·s, and the like; according to the present invention, a stable O/W emulsion can be manufactured by using generally used polyvinyl acetate of which a degree of saponification is 70 to 96 mol%, irrespectively of the molecular weight and the viscosity. From the point of view of preparations and feel when used, a partially saponified polyvinyl alcohol of which a degree of saponification is 78 to 96 mol% is preferable, and a partially saponified polyvinyl alcohol of which a degree of saponification is 85 to 90 mol% is particularly preferable. Polyvinyl alcohol of which a degree of saponification is 97 mol% or higher, which is referred to as completely saponified polyvinyl alcohol, is generally distinguished from partially saponified polyvinyl alcohol in the present invention.

The amount of partially saponified polyvinyl alcohol is 0.1 to 4.0 mass% of the total amount of the composition, preferably 0.2 to 3.0 mass%, in preparations. The partially saponified polyvinyl alcohol can be adjusted to an appropriate amount on the basis of other components and the purpose of use; in the case of a cream or lotion for external application, for example for skin, it is preferable in such an amount that the partially saponified polyvinyl alcohol is 0.5 to 2.0 mass% of the total amount of the product.

The term "carboxyvinyl polymer" in the present invention means a copolymer of acrylic acid, and includes several types having dif ferent viscosities, such that the viscosity of a 0.5% aqueous solution is, for example, 4,000 to 10,000 mPa·s, 40,000 to 60,000 mPa·s and the like, from which an appropriate type can be selected on the basis of the purpose of use according to the present invention. In the case of a cream for external application, for example, for the skin, it is preferable to use mainly a type having a high viscosity, and in the case of a lotion, it is preferable to use mainly a type having a low viscosity.

The amount of carboxyvinyl polymer is 0.1 to 3.0 mass% of the total amount of the composition, preferably 0.3 to 2.0 mass%, in preparations; thus, a highly stable O/W emulsion having excellent feel when used can be obtained.

The amount of an oil component mixed is 40 wt% or less of the total amount of the composition, and 60 wt% or more of the oil component is liquid oil. In the case where the oil component exceeds 40 wt% of the total amount of the composition, the oil component becomes separated and an emulsion cannot be prepared. Furthermore, in the case where the ratio of the liquid oil in the oil component is lower than 60 wt%, the properties become poor and the stability is decreased. It is more preferable for the oil component to be 35 wt% or less of the total amount of the composition, in preparations.

In addition, the term "oil component" in the present invention means any of liquid oil, a solid fat or a mixture of these, preferably liquid oil. The term "Liquid oil" means either nonpolar or polar oil which is in a liquid state at 25°C. The liquid oil is preferably polar liquid oil. The polar liquid oil is preferably synthetic oil of which an IOB value (inorganic organic balance value) is 0.1 or higher or a vegetable oil of which a main component is triglyceride fatty acid.

Here, the IOB value is a numeric value determined in such a manner that an organic value and an inorganic value are found on the basis of [Yoshio Koda, "Organic Concept Diagram-Basics and Application," Sankyo Publishing Co., Ltd. (1984)] to divide the inorganic value by the organic value.

As for the oil component, examples of nonpolar liquid oils include liquid paraffin and synthetic squalane; examples of polar liquid oils include medium chain triglyceride fatty acids, octyldodecyl myristate, cetyl isooctanoate, isopropyl myristate, diglyceryl triisostearate, triglyceryl (caprylate/caprate), triglyceryl 2-ethylhexanoate, diisopropyl sebacate, tri 2-ethylhexyl citrate, diisopropyl adipate, propylene glycol monocaprylate, diethyl sebacate, 2-hexyldecyl isostearate, isopropyl palmitate, isopropyl linoleate, ethyl linoleate, triethylhexanoin, propylene glycol dicaprylate, butyl ethyl propane diol ethylhexanoate, isocetyl myristate, butyl myristate, cetyl ethylhexanoate, ethyl oleate, ethylhexyl palmitate, isopropyl isostearate, ethylhexyl stearate, octyldodecyl neopentanoate, propylene glycol dicaprate, pentaerythrityl tetraethylhexanoate, propylene glycol dicaprylate, trimethylolpropane triethylhexanoate, trimethylolpropane triisostearate, dibutyloctylsebacate, diisostearylmalate, isocetyl stearate, isostearyl palmitate, octyldodecanol, hexyldodecanol, oleyl alcohol, isostearyl alcohol, olive oil, almond oil, safflower oil, castor oil, corn oil, avocado oil, persic oil, candlenut oil, grape seed oil, pistachio nut oil, hazelnut oil, macadamia nut oil, meadowfoam oil, rosehip oil, sunflower oil, soybean oil, jojoba oil and oleic acid. Examples of solid fats include solid paraffin, white vaseline, myristyl myristate, cetyl palmitate, stearyl alcohol, cetanol, cetostearyl alcohol, behenyl alcohol, stearic acid, myristic acid, hard fat, jojoba wax and cacao oil can be cited as.

The oil component is particularly preferably a synthetic liquid oil of which an IOB value is 0.1 ormore, selected fromcetyl isooctanoate, isopropyl myristate, diglyceryl triisostearate, triglyceryl (caprylate/caprate), tri 2-glyceryl ethylhexanoate, diisopropyl sebacate, tri 2-ethylhexyl citrate, diisopropyl adipate, propylene glycol monocaprylate, diethyl sebacate, 2-hexidecyl isostearate, isopropyl palmitate, isopropyl linoleate, ethyl linoleate, triethylhexanoin, propylene glycol dicaprylate, butyl ethyl propane diol ethylhexanoate, isocetyl myristate, butyl myristate, cetyl ethylhexanoate, ethyl oleate, ethylhexyl palmitate, isopropyl isostearate, ethylhexyl stearate, octyldodecyl neopentanoate, propylene glycol dicaprylate, pentaerythrityl tetraethylhexanoate, propylene glycol dicaprylate, trimethylolpropane triethylhexanoate, trimethylolpropane triisostearate, dibutyloctyl sebacate and diisostearyl malate, or a liquid vegetable oil of which the main component is triglyceride fatty acid, selected from olive oil, almond oil, safflower oil, castor oil, corn oil, avocado oil, persic oil, candlenut oil, grape seed oil, pistachio nut oil, hazelnut oil, macadamia nut oil, meadowfoam oil, rosehip oil, sunflower oil and soybean oil.

One or more types of these oil components may be combined for use.

The pH of the O/W emulsion of the invention is preferably adjusted to 3.5 to 8.5. In the case where the value of the pH is less than 3.5, stability in emulsion becomes poor, failing to obtain a target O/W emulsion. In the case where the O/W emulsion of the invention used for the skin has a pH exceeding 8.5, there is a concern that the skin may be stimulated, and therefore, the O/W emulsion is not appropriate for the skin. From the point of view of preparations, the pH is more preferably adjusted to 4.5 to 8.0.

Although a pH adjusting agent is not particularly limited, basic compounds which are mixed in conventional pharmaceutical products or cosmetics, can be used. Examples of the pH adjusting agent include diisopropanolamine, triisopropanolamine, sodium hydroxide, monoethanolamine, diethanolamine, triethanolamine, triethylamine, potassium hydroxide and sodium citrate. When the pH is adjusted, one or more types of these pH adjusting agents can be combined for use.

The "composition for external application" according to the present invention can be used in a general form, such as a cream, a lotion, an ointment, a gel, a spray and the like, in the case where the O/W emulsion is applied in portions of the skin, the mucous membrane, the eyes or the like, and thus, can be used for pharmaceutical products, cosmetics, quasi-drugs and the like.

Furthermore, in the case where a polyol is mixed into the O/W emulsion according to the invention, the excellent feel of this emulsion when used can be preserved. The types of polyols which can be used in the invention are not particularly limited, and examples of the polyols include 1,3-butylene glycol, propylene glycol, dipropylene glycol, glycerin and polyethylene glycol. When a polyol is mixed into the O/W emulsion according to the invention, one or more types from among these polyols can be combined in a mixture.

The phrase "includes substantially no surfactant" in the present invention means that no surfactant is mixed or such an amount that the surfactant does not have any substantial effects is mixed.

Here, the term "surfactant" means a compound which has a hydrophilic group and a hydrophobic group so as to be dissolvable in water, and thus greatly changes the properties in terms of surface tension and the like, and at the same time, forms association, such as micelles or the like, in water.

The O/W emulsion according to the invention can be manufactured in accordance with general methods for manufacturing emulsions. For example, a carboxyvinyl polymer is dispersed in purified water and heated, partially saponified vinyl alcohol is separately dissolved in heated purified water, and then, the two aqueous solutions are mixed together to form a water phase. Meanwhile, an oil component (an appropriate amount of polyols can be added if necessary) is heated to form an oil phase. The oil phase is added to the water phase, and purified water is further added thereto. The mixture is stirred until it becomes uniform, followed by being cooled to room temperature while stirring, thereby to obtain a target O/W emulsion. An appropriate amount of a pH adjusting agent is dissolved in the oil phase or the purified water which is added at the end, and thus mixed.

The thus prepared O/W emulsion product can be preserved stably over a long period of time, and it is not necessary to mix a surfactant; therefore, the emulsion stimulates little when used on the skin. Furthermore, the emulsion spreads smoothly, and excellent feel continues when used, even after application.

In addition, active components and various types of basic components which can be contained in pharmaceutical products, quasi-drugs and cosmetics may be added to the 0/W emulsion according to the invention, as long as the target effects are not lost.

Examples of the active components include anti-inflammatory agents (such as indomethacin, piroxicam, diclofenac sodium, felbinac, hydrocortisone acetate, dexamethasone acetate and the like), analgesics, antihistaminic agents (such as diphenhydramine, chloropheniramine maleate, isothipendyl hydrochloride and the like), local anesthetics (such as lidocaine, procaine, dibucaine, lidocaine hydrochloride, procaine hydrochloride, dibucaine hydrochloride or the like), repairing agents for tissue, antipruritic agents (such as crotamiton or the like), urea, moisturizing agents (such as hyaluronic acid, ceramide and the like), refrigerants (suchasmenthol, camphor agents and the like), vitamins and derivatives thereof (such as vitamin A, vitamin B, vitamin C, vitamin E and the like), angiotonic (such as tetrahydrozoline hydrochloride, phenylephrine hydrochloride and the like), whitening agents, antifungals, anti-allergy agents, antiviral agents, antifungal agents (such as miconazole nitrate, terbinafine hydrochloride, bifonazole, neticonazole hydrochloride, butenafine hydrochloride, liranaftate, and luliconazole), oxygen removing agents, ultraviolet ray absorbing agents and ultraviolet ray scattering agents.

Examples of the base components include dissolution aiding agents, such as low class alcohols (ethanol, isopropanol and the like), hydrocarbons, wax components, antioxidants (such as dibutyl hydroxy toluene), emulsion stabilizers, gelling agents (such as methyl cellulose, hydroxyethyl cellulose, hydroxypropyl cellulose, hydroxy propyl methyl cellulose, sodium alginate, xanthan gum, and polyvinylpyrrolidone), thickeners, adhesives, extracts from various types of animals and plants, antiseptics (such as parahydroxybenzoates benzyl alcohol, and phenoxyethanol), chelating agents (such as sodium edetate), fragrances, pigments and liquefied gases.

Next, although the present invention will be described in more detail based on examples, the invention is not limited to these examples.

### [Examples]

In the following, the present invention will be described in more detail with reference to examples, comparative examples and test examples. Unless otherwise stated, Carbopol 980, manufactured by Noveon Inc., was used as the carboxyvinyl polymer, Panacet 810, manufactured by NOF corporation, was used as the medium chain triglyceride fatty acid, and Gohsenol EG-05, manufactured by the Nippon Synthetic Chemical Industry Co., Ltd., was used as the partially saponified polyvinyl alcohol (degree of saponification: 86.5 to 89.0 mol%).

**Example 1**

| | |
|---|---|
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water pH 5.1 | 100 w/w% in total |

0.5 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and (an appropriate amount of) diisopropanolamine was dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 2**

| | |
|---|---|
| Hexyldecanol | 4 w/w% |
| isopropyl myristate | 12 w/w% |
| carboxyvinyl polymer | 0.8 w/w% |
| partially saponified polyvinyl alcohol (degree of saponification: 86.5 to 89.0 mol%) | 1.2 w/w% |
| glycerin | 5 w/w% |
| sodium hydroxide | appropriate amount |
| purified water | 100 w/w% in total |
| pH | 4.8 |

0.8 g of a carboxyvinyl polymer was dispersed in 40 g of purified water and heated to approximately 70°C so that a solution was gained. Further, 1. 2 g of partially saponified polyvinyl alcohol was dissolved in 20 g of purified water heated to approximately 70°C to obtain an aqueous solution. The two aqueous solutions were mixed together to formawaterphase. Separately, a mixture of 12 g of isopropyl myristate, 4 g of hexyldecanol and 5 g of glycerin was heated to approximately 70°C to form an oil phase. The oil phase was added to the water phase and the mixture was stirred, followed by addition of the remaining purified water in which (an appropriate amount of) sodium hydroxide was dissolved. Further, the mixture was cooled to room temperature while stirring so that it became uniform, thereby obtaining a cream.

**Example 3**

| | |
|---|---|
| isopropyl myristate | 10 w/w% |
| stearyl alcohol | 2 w/w% |
| carboxyvinyl polymer | 0.5 w/w% |
| partially saponified polyvinyl alcohol (degree of saponification: 86.5 to 89.0 mol%) | 0.8 w/w% |
| triethanolamine | appropriate amount |
| purified water | 100 w/w% in total |
| pH | 6.0 |

0.5 g of a carboxyvinyl polymer was dispersed in 25 g of purified water and heated to approximately 70°C so that a solution was gained. Further, 0.8 g of partially saponified polyvinyl alcohol was dissolved in 25 g of purified water heated to approximately 70°C to obtain an aqueous solution. The two aqueous solutions were mixed together to formawaterphase. Separately, amixtureof 10 g of isopropyl myristate and 2 g of stearyl alcohol was heated to approximately 70°C to form an oil phase. The oil phase was added to the water phase and the mixture was stirred, followed by addition of the remaining purified water in which (an appropriate amount of) triethanolamine was dissolved. Further, the mixture was cooled to room temperature while stirring so that it became uniform, thereby obtaining a cream.

**Example 4**

| | |
|---|---|
| medium chain triglyceride fatty acid | 12 w/w% |
| squalane | 2 w/w% |
| carboxyvinyl polymer | 0.5 w/w% |
| partially saponified polyvinyl alcohol (degree of saponification: 86.5 to 89.0 mol%) | 1 w/w% |
| polyethylene glycol 400 | 12 w/w% |
| diisopropanolamine | appropriate amount |
| purified water | 100 w/w% in total |
| pH | 7.1 |

0.5 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 12 g of medium chain triglyceride fatty acid, 2 g of squalane and 12 g of polyethylene glycol 400 was heated to approximately 70°C, and (an appropriate amount of) diisopropanolamine was dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 5**

| | |
|---|---|
| medium chain triglyceride fatty acid | 15 w/w% |
| carboxyvinyl polymer | 0.5 w/w% |
| hydroxy propyl methyl cellulose | 0.05 w/w% |
| partially saponified polyvinyl alcohol (degree of saponification: 86.5 to 89.0 mol%) | 1.2 w/w% |
| 1,3-butylen glycol | 10 w/w% |
| diisopropanolamine | appropriate amount |
| purified water | 100 w/w% in total |
| pH | 5.5 |

0.5 g of a carboxyvinyl polymer was dispersed in 25 g of purified water and heated to approximately 70°C so that a solution was gained. Further, 1. 2 g of partially saponified polyvinyl alcohol was dissolved in 15 g of purified water heated to approximately 70°C to obtain an aqueous solution. Furthermore, 0.05 g of hydroxy propyl methyl cellulose was swollen with 10 g of purified water heated to 70°C, to obtain an aqueous solution. The three aqueous solutions were mixed together to form a water phase. Separately, a mixture of 15 g of medium chain triglyceride fatty acid and 10 g of 1,3-butylen glycol was heated to approximately 70°C, and (an appropriate amount of) diisopropanolamine was dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 6**

| | |
|---|---|
| medium chain triglyceride fatty acid | 10 w/w% |
| carboxyvinyl polymer | 0.1 w/w% |

| (Carbopol 980*) | |
|---|---|
| carboxyvinyl polymer | 0.2 w/w% |

| (Carbopol 981**) | |
|---|---|
| partially saponified polyvinyl alcohol (degree of saponification: 86.5 to 89.0 mol%) | 1 w/w% |
| 1,3-butylen glycol | 10 w/w% |
| diisopropanolamine | appropriate amount |
| purified water | 100 w/w% in total |
| pH | 5.0 |

| | |
|---|---|
| Carbopol 980*: manufactured by Noveon Inc., viscosity: 40,000 to 60,000 mPa·s (0.5%) Carbopol 981**: manufactured by Noveon Inc., viscosity: 4,000 to 10,000 mPa·s (0.5%) | |

0.3 g in total of two types of carboxyvinyl polymers was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 10 g of medium chain triglyceride fatty acid and 10 g of 1,3-butylen glycol was heated to approximately 70°C, and (an appropriate amount of) diisopropanolamine was dissolved in the mixture to form an oil phase. The oil phase was added to the water phase, and the remaining purified water was further added thereto. The mixture was stirred until it became uniform, followed by being cooled to room temperature while stirring, thereby to form a lotion.

**Example 7**

| | |
|---|---|
| isopropyl methyl phenol | 0.1 w/w% |
| dl-camphor | 0.5 w/w% |
| cetanol | 4 w/w% |
| medium chain triglyceride fatty acid | 11 w/w% |
| carboxyvinyl polymer | 0.7 w/w% |
| partially saponified polyvinyl alcohol (degree of saponification: 86.5 to 89.0 mol%) | 1 w/w% |
| polyethylene glycol 400 | 5 w/w% |
| sodium edetate | 0.1 w/w% |
| diisopropanolamine | appropriate amount |
| purified water | 100 w/w% in total |
| pH | 5.5 |

0.7 g of a carboxyvinyl polymer was dispersed in 35 g of purified water and heated to approximately 70°C so that a solution was gained. Further, 1 g of partially saponified polyvinyl alcohol was dissolved in 20 g of purified water heated to approximately 70°C to obtain an aqueous solution. The two aqueous solutions were mixed together to form a water phase. Separately, a mixture of 11 g of medium chain triglyceride fatty acid and 4 g of cetanol was heated to approximately 70°C, and 0.5 g of dl-camphor was dissolved in the mixture to form an oil phase. Furthermore, 5g of polyethylene glycol 400 was heated to approximately 70°C and 0.1 g of isopropyl methyl phenol and (an appropriate amount of) diisopropanolamine were dissolved to form a polyol phase. The oil phase and the polyol phase were added to the water phase in this order, followed by addition of the remaining purified water in which 0.1 g of sodium edetate was dissolved. The mixture was stirred until it became uniform, followed by being cooled to room temperature while stirring, thereby to form a cream.

**Example 8**

| | |
|---|---|
| dexamethasone acetate | 0.025 w/w% |
| retinol palmitate | 200,000 I.U |
| 1-menthol | 0.25 w/w% |
| medium chain triglyceride fatty acid | 12 w/w% |
| liquid paraffin | 1 w/w% |
| carboxyvinyl polymer | 0.6 w/w% |
| partially saponified polyvinyl alcohol (degree of saponification: 86.5 to 89.0 mol%) | 1.2 w/w% |
| 1,3-butylene glycol | 8 w/w% |
| parahydroxy ethyl benzoate | 0.1 w/w% |
| parahydroxy propyl benzoate | 0.1 w/w% |
| dibutyl hydroxy toluene | 0.1 w/w% |
| sodium hydroxide | appropriate amount |
| purified water | 100 w/w% in total |
| pH | 5.0 |

0.6 g of a carboxyvinyl polymer was dispersed in 30 g of purified water and heated to approximately 70°C so that a solution was gained. Further, 1. 2 g of partially saponified polyvinyl alcohol was dissolved in 25 g of purified water heated to approximately 70°C to obtain an aqueous solution. The two aqueous solutions were mixed together to form a water phase. Separately, a mixture of 12 g of medium chain triglyceride fatty acid and 1 g of liquid paraffin was heated to approximately 70°C, and 200,000 I.U of retinol palmitate, 0.25 g of 1-menthol, 0.1 g of dibutyl hydroxy toluene, 0.1 g of parahydroxy ethyl benzoate and 0.1 g of parahydroxy propyl benzoate were dissolved in the mixture to form an oil phase. Furthermore, 8 g of 1, 3-butylene glycol was heated to approximately 70°C, and 0.025 g of dexamethasone acetate was dissolved therein to form a polyol phase. The oil phase and the polyol phase were added to the water phase in this order, followed by addition of the remaining purified water in which (an appropriate amount of) sodium hydroxide was dissolved. The mixture was stirred until it became uniform, followed by being cooled to room temperature while stirring, thereby to form a cream.

**Example 9**

| | |
|---|---|
| lidocaine hydrochloride | 1 w/w% |
| medium chain triglyceride fatty acid | 13 w/w% |
| carboxyvinyl polymer | 0.75 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water | 100 w/w% in total |
| pH | 6.5 |

0.75 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 1 g of lidocaine hydrochloride and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 10**

| | |
|---|---|
| dibucaine | 0.5 w/w% |
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water pH 6.9 | 100 w/w% in total |

0.5 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 0.5 g of dibucaine and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 11**

| | |
|---|---|
| indomethacin | 1 w/w% |
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water pH 6.3 | 100 w/w% in total |

0.5 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 1 g of indomethacin and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 12**

| | |
|---|---|
| diphenhydramine | 0.5 w/w% |
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water | 100 w/w% in total |
| pH | 6.8 |

0.5 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 0.5 g of diphenhydramine and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 13**

| | |
|---|---|
| miconazole nitrate | 1 w/w% |
| medium chain triglyceride fatty acid | 13 w/w% |
| carboxyvinyl polymer | 0.75 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water | 100 w/w% in total |
| pH | 6.6 |

0.75 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 1 g of miconazole nitrate and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 14**

| | |
|---|---|
| procaine hydrochloride | 1 w/w% |
| medium chain triglyceride fatty acid | 13 w/w% |
| carboxyvinyl polymer | 0.75 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water pH 6.9 | 100 w/w% in total |

0.75 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 1 g of procaine hydrochloride and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 15**

| | |
|---|---|
| terbinafine hydrochloride | 1 w/w% |
| medium chain triglyceride fatty acid | 13 w/w% |
| carboxyvinyl polymer | 0.8 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water pH 6.0 | 100 w/w% in total |

0.8 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 1 g of terbinafine hydrochloride and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 16**

| | |
|---|---|
| ubidecarenone | 0.03 w/w% |
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water | 100 w/w% in total |
| pH | 5.3 |

0.5 g of carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and 0.03 g of ubidecarenone and (an appropriate amount of) diisopropanolamine were dissolved in the mixture to form an oil phase. The oil phase was added to the water phase, and the remaining purified water was further added thereto. The mixture was stirred until it became uniform, followed by being cooled to room temperature while stirring, thereby to form a cream.

**Example 17**

| | |
|---|---|
| sodium hyaluronate | 0.01 w/w% |
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water | 100 w/w% in total |
| pH | 5.3 |

0.5 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and (an appropriate amount of) diisopropanolamine was dissolved in the mixture to form an oil phase. The oil phase was added to the water phase, followed by addition of the remaining purified water in which 0.01 g of sodium hyaluronate was dissolved. The mixture was stirred until it became uniform, followed by being cooled to room temperature while stirring, thereby to form a cream.

**Example 18**

| | |
|---|---|
| N-stearoyl phytosphingosine | 0.1 w/w% |
| medium chain triglyceride fatty acid | 10 w/w% |
| propylene glycol monocaprylate | 10 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water | 100 w/w% in total |
| pH | 5.5 |

0.5 g of a carboxyvinyl polymer was dispersed in 25 g of purified water and heated to approximately 70°C so that a solution was gained. Further, 1 g of partially saponified polyvinyl alcohol was dissolved in 25 g of purified water heated to approximately 70°C to obtain an aqueous solution. The two aqueous solutions were mixed together to form a water phase. Separately, a mixture of 10 g of medium chain triglyceride fatty acid and 10 g of propylene glycol monocaprylate was heated to approximately 70°C, and 0.1 g of N-stearoyl phytosphingosine was dissolved in the mixture to form an oil phase. 10 g of 1, 3-butylene glycol was heated to approximately 70°C and (an appropriate amount of) diisopropanolamine was dissolved to form a polyol phase. The oil phase was added to the water phase, and the polyol phase and the remaining purified water were further added thereto. The mixture was stirred until it became uniform, followed by being cooled to room temperature while stirring, thereby to form a cream.

### Test Example 1 (Sample Test 1: Carboxyvinyl Polymer Modified/Partially Saponified PolyvinylAlcoholModified Sample Test)

0.5 g of a carboxyvinyl polymer was dispersed in 25 g of purified water and heated to approximately 70°C so that a solution was gained. Further, 1 g of partially saponified polyvinyl alcohol (degree of saponification is 86.5 mol% to 89.0 mol%) was dissolved in 25 g of purified water heated to approximately 70°C to obtain an aqueous solution. The two aqueous solutions were mixed together to form a waterphase. Separately, a mixture of 13 g of medium chain triglyceride fatty acid and 10 g of 1, 3-butylene glycol was heated to approximately 70°C, and (an appropriate amount of) diisopropanolamine was dissolved in the mixture to form an oil phase. The oil phase was added to the water phase, and the remaining purified water was further added thereto. The mixture was stirred until it became uniform, followed by being cooled to room temperature while stirring, thereby to form a cream. The cream was used as a base formulation. The pH was adjusted to 5 to 7.

Next, the following modified formulations were prepared.

### (1) Modified Formulation of Partially Saponified Polyvinyl Alcohol (degree of saponification is 86.5 to 89.0 mol%)

Modified formulations obtained by replacing the partially saponified polyvinyl alcohol in the base formulation with sodium carboxymethyl cellulose, hydrophobic hydroxypropyl methyl cellulose, hydroxypropyl cellulose, hydroxyethyl cellulose, sodium alginate and polyvinyl alcohol (degree of saponification is not less than 97.0 mol%), respectively, were prepared to form a cream in the same manner as the base formulation.

### (2) Modified Formulation of Carboxyvinyl Polymer

Modified formulations obtained by replacing the carboxyvinyl polymer in the base formulation with sodium carboxymethyl cellulose, hydrophobic hydroxypropyl methyl cellulose, xanthan gum, sodium alginate, hydroxyethyl cellulose, hydroxypropyl cellulose and polyvinylpyrrolidone, respectively, were prepared to form a cream in the same manner as the base formulation.

### (3) Omission Formulation

Respective modified formulations obtained by omitting the partially saponified polyvinyl alcohol or carboxyvinyl polymer in the base formulation were prepared in the same manner as the base formulation.

Whether or not it was possible to prepare a uniform product having excellent feel when used in accordance with these modified formulations was confirmed. Visual confirmation was carried out by three people. The results are shown in Table 1. × indicates failure of uniform preparation.

**[Table 1]**

| EXAMINED FORMULATION | OMISSION | MODIFICATION | |
|---|---|---|---|
| | | SODIUM CARBOXYMETHYL CELLULOSE | × |
| PARTIALLY SAPONIFIED POLYVINYL ALCOHOL (DEGREE OF SAPONIFICATION: 86.5 TO 89.0 mol%) | | HYDROPHOBIC HYDROXYPROPYL METHYL CELLULOSE | × |
| | × | HYDROXYPROPYL CELLULOSE | × |
| | | HYDROXYETHYL CELLULOSE | × |
| | | SODIUM ALGINATE | × |
| | | POLYVINYL ALCOHOL (DEGREE OF SAPONIFICATION: 97 mol % OR MORE) | × |
| | | SODIUM CARBOXYMETHYL CELLULOSE | × |
| | | HYDROPHOBIC HYDROXYPROPYL METHYL CELLULOSE | × |
| | | XANTHAN GUM | × |
| CARBOXYVINYL POLYMER | × | SODIUM ALGINATE | × |
| | | HYDROXYETHYL CELLULOSE | × |
| | | HYDROXYPROPYL CELLULOSE | × |
| | | POLYVINYLPYRROLIDONE | × |

### Test Example 2 (Sample Test 2: Oil Component Modified Sample Test)

0.5 g of a carboxyvinyl polymer was dispersed in 25 g of purified water and heated to approximately 70°C so that a solution was gained. Further, 1 g of partially saponified polyvinyl alcohol (degree of saponification is 86. 5 to 89.0 mol%) was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and (an appropriate amount of) diisopropanolamine was dissolved in the mixture to form an oil phase. The oil phase was added to the water phase, and the remaining purified water was further added thereto. The mixture was stirred until it became uniform, followed by being cooled to room temperature while stirring, thereby to form a cream. The cream was used as a base formulation. The pH was adjusted to 5 to 7.

### (1) Modified Formulation of Medium Chain Triglyceride Fatty Acid

Respective formulations obtained by replacing the medium chain triglyceride fatty acid in the base formulation with paraffin, white Vaseline (semi-solid), liquid paraffin, synthetic squalane, cetyl palmitate, myristyl myristate, behenyl alcohol, cetanol, batyl alcohol, octyldodecyl myristate, cetyl isooctanoate, isopropyl myristate, diglyceryl triisostearate, triglyceryl (caprylate/caprate), triglyceryl 2-ethylhexanoate, diisopropyl sebacate, tri 2-ethylhexyl citrate, diisopropyl adipate, propylene glycolmonocaprylate, olive oil, almond oil and castor oil were prepared to form a cream in the same manner as the base formulation.

### (2) Omission Formulation

A modified formulation obtained by omitting the medium chain triglyceride fatty acid in the base formulation was prepared in the same manner as the base formulation.

Whether or not it was possible to prepare a uniform product having excellent feel when used in accordance with these modified formulations was confirmed. Visual confirmation was carried out by three people. The results are shown in Table 2. In the row "65 °C/2W", the properties after passage of 65°C 2W were confirmed.

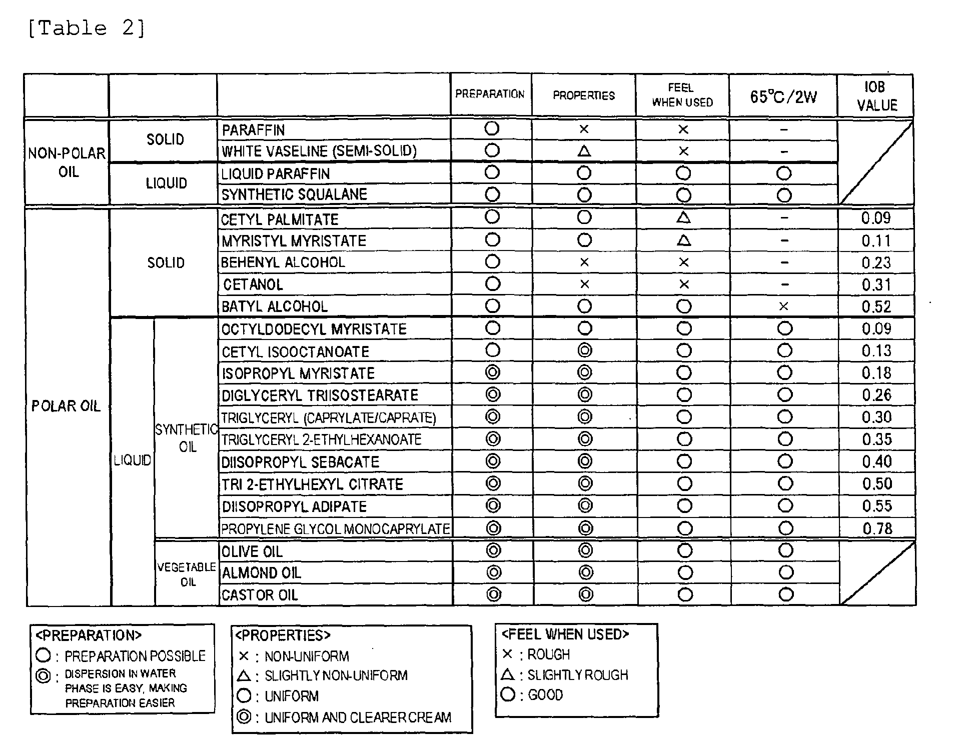

In accordance with the omission formulation, no emulsion composition could be prepared.

### Test Example 3 (Sample Test 3: pHAdjusting Agent Modified/Polyol Modified Sample Test)

0.5 g of a carboxyvinyl polymer was dispersed in 25 g of purified water and heated to approximately 70°C so that a solution was gained. Further, 1 g of partially saponified polyvinyl alcohol (degree of saponification is 86.5 to 89.0 mol%) was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and (an appropriate amount of) diisopropanolamine was dissolved in this mixture to form an oil phase. The oil phase was added to the water phase, and the remaining purified water was further added thereto. The mixture was stirred until it became uniform, followed by being cooled to room temperature while stirring, thereby to form a cream. The cream was used as a base formulation. The pH was adjusted to 5 to 7.

### (1) Modified Formulation of Diisopropanolamine

Respective formulations obtained by replacing the diisopropanolamine in the base formulation with sodium hydroxide, triisopropanolamine, monoethanolamine, diethanolamine, triethanolamine and triethylamine were prepared to form a cream in the same manner as the base formulation.

### (2) Modified Formulation of 1,3-butylene glycol

Respective formulations obtained by replacing the 1, 3-butylene glycol in the base formulation with propylene glycol, glycerin, polyethylene glycol 400 and polyethylene glycol 4000 were prepared to form a cream in the same manner as the base formulation.

### (3) Omission Formulation

Modified formulations obtained by omitting the diisopropanolamine or 1,3-butylene glycol in the base formulation were prepared to form a cream in the same manner as the base formulation.

Whether or not it was possible to prepare a uniform product having excellent feel when used in accordance with these modified formulations was confirmed. Visual confirmation was carried out by three people. The results are shown in Table 3. o in the table indicates that a uniform cream having excellent feel when used was prepared. Δ indicates that though a uniform product having excellent feel when used was prepared, it was a product with slightly poor appearance.

**[Table 3]**

| | | OMISSION | MODIFICATION | |
|---|---|---|---|---|
| | | | SODIUM HYDROXIDE | ○ |
| | | | TRIISOPROPANOLAMINE | ○ |
| pH ADJUSTING AGENT | DIISOPROPANOLAMINE | | TRIETHYLAMINE | ○ |
| | | Δ | TRIETHANOLAMINE | ○ |
| | | | DIETHANOLAMINE | ○ |
| | | | MONOETHANOLAMINE | ○ |
| | | | PROPYLENE GLYCOL | ○ |
| POLYOL | 1,3-BUTYLENE GLYCOL | | GLYCERIN | ○ |
| | | ○ | POLYETHYLENE GLYCOL 400 | ○ |
| | | | POLYETHYLENE GLYCOL 4000 | ○ |

### Test Example 4 (Sample Test 4: Mixture Amount Modifying Test)

0.5 g of a carboxyvinyl polymer was dispersed in 25 g of purified water and heated to approximately 70°C so that a solution was gained. Further, 1 g of partially saponified polyvinyl alcohol (degree of saponification is 86.5 to 89.0 mol%) was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and (an appropriate amount of) diisopropanolamine was dissolved in this mixture to form an oil phase. The oil phase was added to the water phase, and the remaining purified water was further added thereto. The mixture was stirred until it became uniform, followed by being cooled to room temperature while stirring, thereby to form a cream. The cream was used as a base formulation. The pH was adjusted to 5 to 7.

Next, the following mixture amount modified formulations were prepared.

### (1) Partially Saponified Polyvinyl Alcohol (degree of saponification: 86.5 to 89.0 mol%) Mixture Amount Modifying Test

Formulations obtained by modifying the mixture amount of the partially saponified polyvinyl alcohol (degree of saponification: 86.5 to 89.0 mol%) in the base formulation were prepared to form a cream in the same manner as the base formulation.

In the partially saponified polyvinyl alcohol mixture amount modified formulations, whether or not it was possible to prepare a uniform product having excellent feel when used was confirmed. Visual confirmation was carried out by three people. The results are shown in Table 4-1. o in the row "preparation" in the table indicates that a uniform product could be prepared, Δ indicates that slight separation was observed, and × indicates that a uniform product could not be prepared. o in the row "feel when used" indicates that the product spread smoothly and had excellent feel when used, and Δ indicates that the product had a slightly sticky feel.

In the row "65°C/2W" the properties after passage of 65°C 2W were confirmed. o indicates that there was no change immediately after preparation.

**[Table 4-1]**

| POLYVINYL ALCOHOL MIXTURE AMOUNT% (DEGREE OF SAPONIFICATION 86.5 TO 98.0 mol%) | 0.025 | 0.05 | 0.1 | 0.2 | 0.3 | 0.4 | 0.5 | 2.0 | 3.0 | 4.0 |
|---|---|---|---|---|---|---|---|---|---|---|
| PREPARATION | × | Δ | ○ | ○ | ○ | ○ | ○ | ○ | ○ | ○ |
| FEEL WHEN USED | - | - | ○ | ○ | ○ | ○ | ○ | ○ | ○ | Δ |
| 65°C/2W | - | - | ○ | ○ | ○ | ○ | ○ | ○ | ○ | - |

### (2) Carboxyvinyl Polymer Mixture Amount Modifying Test

Formulations obtained by modifying the mixture amount of the carboxyvinyl polymer in the base formulation were prepared to form a cream in the same manner as the base formulation.

In the carboxyvinyl polymer mixture amount modified formulations, whether or not it was possible to prepare a uniform product having excellent feel when used was confirmed. Visual confirmation was carried out by three people. The results are shown in Table 4-2. o in the row "preparation" in the table indicates that a uniformproduct could be prepared, Δ indicates that slight separation was observed, and × indicates that a uniform product could not be prepared. o in the row "feel when used" indicates that the product spread smoothly and had excellent feel when used, and Δ indicates that the product had a slightly sticky feel.

In the row "65°C/2W" the properties after passage of 65°C 2W were confirmed. O indicates that the product was uniform, without any change immediately after preparation, and Δ indicates that the product became slightly non-uniform.

**[Table 4-2]**

| TYPE | MIXTURE AMOUNT % | 0.1 | 0.2 | 0.3 | 0.4 | 0.5 | 2.0 | 3.0 | 4.0 |
|---|---|---|---|---|---|---|---|---|---|
| LOW VISCOSITY (4000~10000m Pa·s (0.5%)) | PREPARATION | Δ | Δ | ○ | ○ | ○ | ○ | ○ | ○ |
| | FEEL WHEN USED | - | - | ○ | ○ | ○ | ○ | ○ | Δ |
| | 65°C/2W | - | - | ○ | ○ | ○ | ○ | Δ | Δ |
| | MIXTURE AMOUNT % | 0.05 | 0.1 | 0.15 | 0.2 | 0.3 | 1.0 | 2.0 | 3.0 |
| HIGH VISCOSITY (40000~60000mPa·s(0.596)) | PREPARATION | Δ | ○ | ○ | ○ | ○ | ○ | ○ | ○ |
| | FEEL WHEN USED | - | ○ | ○ | ○ | ○ | ○ | ○ | Δ |
| | 65°C/2W | - | ○ | ○ | ○ | ○ | ○ | ○ | Δ |

### (3) Oil Mixture Amount Modifying Test

Formulations obtained by modifying the mixture amount of the oil component in the base formulation were prepared to form a cream in the same manner as the base formulation.

In the oil component mixture amount modified formulations, whether or not it was possible to prepare a uniform product having excellent feel when used was confirmed. Visual confirmation was carried out by three people. The results are shown in Table 4-3. o in the row "preparation/feel when used" in the table indicates that a uniform product could be prepared and the product spread smoothly and had excellent feel when used. Δ indicates that slight separation was observed and the product had a slightly sticky feel. × indicates that a uniform product could not be prepared.

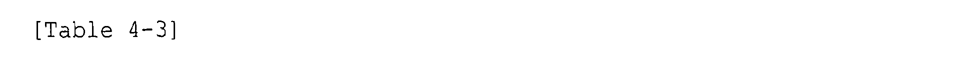

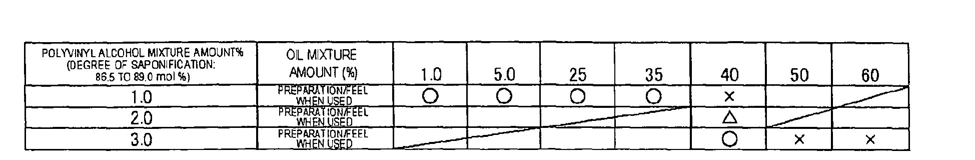

As a result of the 65°C/2W passage changing test, there was no change in the properties of all products having o in all the items.

### Test Example 5 (Sample Test 5: pH Test)

0.5 g of a carboxyvinyl polymer was dispersed in 25 g of purified water and heated to approximately 70°C so that a solution was gained. Further, 1 g of partially saponified polyvinyl alcohol (degree of saponification is 86.5 mol% to 89.0 mol%) was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1, 3-butylene glycol was heated to approximately 70°C, and (an appropriate amount of) diisopropanolamine was dissolved in this mixture to form an oil phase. The oil phase was added to the water phase, and the remaining purified water was further added thereto. The mixture was stirred until it became uniform, followed by being cooled to room temperature while stirring, thereby to form a cream. The cream was used as a base formulation.

Next, the pH of the products was changed by changing the amount of the diisopropanolamine mixed.

In these formulations, whether or not it was possible to prepare a uniform product having excellent feel when used was confirmed. Visual confirmation was carried out by three people. The results are shown in Table 5. o in the row "preparation" in the table indicates that a uniform product could be prepared, and × indicates that a uniform product could not be prepared.

In the row "65°C/2W" the properties after passage of 65°C 2W were confirmed. O indicates that the product was uniform, without any change immediately after preparation.

**[Table 5]**

| pH | 3.1 | 3.5 | 4.0 | 5.3 | 5.8 | 6.2 | 6.9 | 7.7 | 8.9 | 9.3 | 9.7 |
|---|---|---|---|---|---|---|---|---|---|---|---|
| PREPARATION | × | ○ | ○ | ○ | ○ | ○ | ○ | ○ | ○ | ○ | ○ |
| 65°C/2W | - | ○ | ○ | ○ | ○ | ○ | ○ | ○ | ○ | ○ | ○ |

### Test Example 6 (Sample Test 6: Test for changing the mixture amount of a liquid oil relative to the total of oil components)

0.5 g of a carboxyvinyl polymer was dispersed in 25 g of purified water and heated to approximately 70°C so that a solution was gained. Further, 1 g of partially saponified polyvinyl alcohol (degree of saponification is 86.5 to 89.0 mol%) was dissolved in 25 g of purified water heated to approximately 70°C to obtain an aqueous solution. The two aqueous solutions were mixed together to form a water phase. Separately, a mixture of 13 g of medium chain triglyceride fatty acid and 10 g of 1,3-butylene glycol was heated to approximately 70°C, and (an appropriate amount of) diisopropanolamine was dissolved in this mixture to form an oil phase. The oil phase was added to the waterphase, and the remainingpurifiedwaterwas further added thereto. The mixture was stirred until it became uniform, followed by being cooled to room temperature while stirring, thereby to form a cream. The cream was used as a base formulation. The pH was adjusted to 5 to 7.

Formulations were prepared to form a cream in the same manner as the base formulation except that, with the total mixture amount of the oil components in the base formulation being fixed, medium chain triglyceride fatty acid was used as a liquid oil, and cetanol being a solid oil was used as the rest of the oil component to change the mixture amount of the liquid oil relative to the total of the oil components.

In these modified formulations, whether or not it was possible to prepare a uniform product having excellent feel when used was confirmed. Visual confirmation was carried out by three people. The results are shown in Table 6. o in the row "preparation" in the table indicates that a uniform product could be prepared. o in the row "properties" in the table indicates that a uniform product could be gained, Δ indicates that the product was slightly non-uniform, and × indicates that the product was non-uniform. o in the row "feel when used" indicates that the product had excellent feel when used, Δ indicates that the product had a slightly rough feel, and × indicates that the product had a rough feel.

In the row "65°C/2W", the properties after passage of 65°C 2W were confirmed. o indicates that the product was uniform, without any change immediately after preparation.

**[Table 6]**

| MIXTURE AMOUNT (%) OF LIQUID OIL RELATNTE TO TOTAL OF OIL COMPONENTS | 10 | 30 | 40 | 50 | 60 | 70 | 90 |
|---|---|---|---|---|---|---|---|
| PREPARATION | ○ | ○ | ○ | ○ | ○ | ○ | ○ |
| PROPERTIES | × | × | Δ | Δ | ○ | ○ | ○ |
| FEEL WHEN USED | × | × | Δ | Δ | ○ | ○ | ○ |
| 65°C/2W | - | - | - | - | ○ | ○ | ○ |

### Test Example 7 (Stability Test)

The properties (appearance, smell, pH, and observation using microscope (emulsion diameter)) immediately after the preparation of the following sample creams (Formulation Example 1 and Formulation Example 2) were observed and measured. In terms of the appearance and the smell, the cream was taken out from an aluminum tube and observed. The pH was measured using a commercially available pH meter. In terms of the observation using a microscope (emulsion diameter), the emulsion diameter in the cream was measured using a commercially available microscope.

Each cream was sealed in an aluminum tube and preserved over a certain period of time under various temperature and humidity conditions, followed by confirmation of the properties. The conditions for change over time were as follows.
Temperature: 65°C, 2 weeks
Temperature: 50°C, 1 month and 2 months
Temperature: 40°C, humidity: 75%, 3 months and 6 months
Temperature: 5°C, 6 months
The results are shown in Table 7-1 (results for Formulation Example 1) and Table 7-2 (results for Formulation Example 2). In both formulation examples, the emulsion diameter, the pH, the appearance and the smell were all preserved stably over a long period of time.

| Formulation Example 1 | |
|---|---|
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| propylene glycol | 10 w/w% |
| diisopropanolamine | appropriate amount |
| purified water | 100 w/w% in total |

| Formulation Example 2 | |
|---|---|
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamine | appropriate amount |
| purified water | 100 w/w% in total |

**[Table 7-1]**

| | | pH | EMULSION DIAMETER | APPEARANCE | SMELL |
|---|---|---|---|---|---|
| IMMEDIATELY AFTER | | 5.4 | 1~15 µm | WHITE CREAM | SMELL OF BASE |
| 65°C | 2W | 5.3 | 1~20 µm | WHITE CREAM | SMELL OF BASE |
| 50°C | 1M | - | 1~15 µm | WHITE CREAM | SMELL OF BASE |
| | 2M | 5.3 | 1~15 µm | WHITE CREAM | SMELL OF BASE |
| 40°C | 3M | 5.3 | 1~15 µm | WHITE CREAM | SMELL OF BASE |
| | 6M | 5.4 | 1~18 µm | WHITE CREAM | SMELL OF BASE |
| 5°C | 6M | 5.4 | 1~18µm | WHITE CREAM | SMELL OF BASE |

**[Table 7-2]**

| | | pH | EMULSION DIAMETER | APPEARANCE | SMELL |
|---|---|---|---|---|---|
| IMMEDIATELY AFTER | | 5.4 | 1~20 µm | WHITE CREAM | SMELL OF BASE |
| 65°C | 2W | 5.3 | 1~20 µm | WHITE CREAM | SMELL OF BASE |
| 50°C | 1M | - | 1~15 µm | WHITE CREAM | SMELL OF BASE |
| | 2M | 5. 3 | 1~20 µm | WHITE CREAM | SMELL OF BASE |
| 40°C | 3M | 5. 3 | 1~18 µm | WHITE CREAM | SMELL OF BASE |
| | 6M | - | 1~18 µm | WHITE CREAM | SMELL OF BASE |

### Test Example 8 (Test of Feel when Used)

### 1. Comparison of Feel when Used with and without Polyol

An appropriate amount of each of the following Formulation Examples 3 (with polyol) and 4 (without polyol) was applied on part of a forearm and the feel when used was compared. Evaluation was made on the basis of the evaluation standard of the following Table 8-1.
The results are shown in Table 8-2. The values in the results are average values for three people who carried out the test. As a result, no significant difference was observed immediately after application, but Formulation Example 4 (without polyols) had an abrasive feel five minutes after application.

| Formulation Example 3 | |
|---|---|
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| propylene glycol | 10 w/w% |
| diisopropanolamine | appropriate amount |
| purified water | 100 w/w% in total |
| pH | 5.3 |

| Formulation Example 4 | |
|---|---|
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| diisopropanolamine | appropriate amount |
| purified water | 100 w/w% in total |
| pH | 5.4 |

| | |
|---|---|
| * omission of polyols was compensated for with water | |

**[Table 8-1]**

| EVALUATION POINTS | FEEL WHEN USED |
|---|---|
| 4 | GOOD |
| 3 | SLIGHTLY GOOD |
| 2 | NORMAL |
| 1 | SLIGHTLY POOR |
| 0 | POOR |

**[Table 8-2]**

| | FORMULATION EXAMPLE 3 | FORMULATION EXAMPLE 4 |
|---|---|---|
| IMMEDIATELY AFTER APPLICATION | 3.7 POINTS | 3.3 POINTS |
| 5 MINUTES AFTER APPLICATION | 3.7 POINTS | 2.7 POINTS |

### 2. Comparison of Feel when Used Between Formulation with partially saponified polyvinyl alcohol and formulation with surfactant

An appropriate amount of each of the following Formulation Example 5 (formulation with partially saponified polyvinyl alcohol) and Formulation Example 4 (formulation with a surfactant) was applied on part of a forearm, and the feel when used was compared. Evaluation was made to see if there was any stickiness.
The test was performed by three people, and the total evaluation is shown in Table 8-3. As a result, the stickiness five minutes after application was less in Formulation Example 5 (formulation with partially saponified polyvinyl alcohol).

| Formulation Example 5 | |
|---|---|
| medium chain triglyceride fatty acid | 13 w/w% |
| carboxyvinyl polymer | 0.5 w/w% |
| partially saponified polyvinyl alcohol (degree of saponification: 86.5 to 89.0 mol%) | 1 w/w% |
| 1,3-butylene glycol | 10 w/w% |
| diisopropanolamineappropriate amount purified water | 100 w/w% in total |
| pH | 5.3 |

| Formulation Example 6 | |
|---|---|
| medium chain triglyceride fatty acid | 13 w/w% |
| carboxyvinyl polymer | 0.5 w/w% |
| polysorbate 60 | 1 w/w% |
| 1,3-butylene glycol | 10 w/w% |
| diisopropanolamine | appropriate amount |
| purified water | 100 w/w% in total |
| pH | 5.2 |

| | |
|---|---|
| * polysorbate 60: nonionic surfactant | |

**[Table 8-3]**

| | | FORMULATION EXAMPLE 5 | FORMULATION EXAMPLE 6 |
|---|---|---|---|
| STICKNESS | IMMEDIATELY AFTER APPLICATION | SLIGHTLY STICKY | SLIGHTLY STICKY |
| | 5 MINUTES AFTER APPLICATION | SMOOTH | SLIGHTLY STICKY (CLAMMY FEEL) |

### [Industrial Applicability]

According to the present invention, it is possible to obtain a highly safe O/W emulsion composition which can be preserved stably over a long period of time and stimulates little when used on the skin. According to the present invention, it is also possible to obtain an O/W emulsion composition where excellent feel continues when used, even after application.

## Claims

1. An O/W emulsion composition, containing:
a) partially saponified polyvinyl alcohol of which a degree of saponification is 70 to 96 mol%;
b) a carboxyvinyl polymer; and
c) 40 wt% or less of an oil component relative to the total amount of the composition, where 60 wt% or more of the oil component is a liquid oil, wherein
d) the pH is 3.5 to 8.5.

2. The O/W emulsion composition according to Claim 1, including substantially no surfactant.

3. The O/W emulsion composition according to Claim 1, wherein the liquid oil is a polar liquid oil.

4. The O/W emulsion composition according to Claim 3, wherein the polar liquid oil is a synthetic oil of which an IOB value is 0.1 or more.

5. The O/W emulsion composition according to Claim 3, wherein the polar liquid oil is a vegetable oil of which a main component is triglyceride fatty acid.

6. The O/W emulsion composition according to any one of Claims 1 to 5, which is a composition for external application.

7. The O/W emulsion composition according to any one of Claims 1 to 6, further containing a polyol.
